# FASCICULE DE BREVET EUROPEEN

(11) **EP 2 184 083 B1**
(45) Date de publication et mention de la délivrance du brevet: **04.03.2015**
(21) Numéro de dépôt: 09290804.5
(22) Date de dépôt: 20.10.2009
(51) Int. Cl.: A61N 1/05, A61B 17/34, A61M 25/02

(54) **Dispositif de fixation transcranien pour sondes de stimulation cérébrale profonde**
Transkraniale Befestigungsvorrichtung für Sonde zur Tiefenstimulation des Gehirns
Transcranial fixing device for deep brain stimulation probes

(30) Priorité: 23.10.2008 FR 0805873
(43) Date de publication de la demande: 12.05.2010
(73) Titulaire: Commissariat à l'Énergie Atomique et aux Énergies Alternatives, 75015 Paris (FR)
(72) Inventeur: Sauter-Starace, Fabien, 38170 Seyssinet-Pariset (FR); Benabid, Alim-Louis, 38240 Meylan (FR); Charles, Raymond, 38430 Saint Jean de Moirans (FR)
(74) Mandataire: Reboussin, Yohann Mickaël Noël

(56) Documents cités:
- US-A- 6 134 477
- US-A1- 2004 034 367
- US-A1- 2005 182 420
- US-B1- 6 321 104
- US-B1- 6 356 792
- US-B1- 7 004 948
- US-B2- 7 302 298

## Description

L'invention porte sur un dispositif de fixation transcranien pour sondes de stimulation cérébrale profonde. L'invention porte également sur un système de stimulation cérébrale profonde comportant un tel dispositif de fixation.

La stimulation cérébrale profonde (« deep brain stimulation » en langue anglaise) est une technique thérapeutique comportant l'implantation d'un appareil médical connu comme stimulateur cérébral, qui envoie des impulsions électriques à des parties spécifiques du cerveau. Par exemple, la stimulation des nucléus de thalamus ou de l'hypothalamus peut être utilisée pour traiter des désordres moteurs tels que des tremblements, provoqués notamment par le syndrome de Parkinson, tandis que la stimulation du cortex cingulaire subgenual est utilisée à titre expérimental pour le traitement de formes particulièrement graves et résistantes au traitement de dépression clinique.

Dans tous les cas, une intervention de stimulation cérébrale profonde comporte l'insertion dans le crâne du patient d'une sonde souple, guidée par une canule et/ou un stylet rigide, jusqu'à ce que la pointe de ladite sonde atteigne la région du cerveau à stimuler. L'utilisation d'un procédé d'imagerie en temps réel (notamment par résonance magnétique nucléaire), couplé avec une exploration électrophysiologique, permet de savoir exactement quand la région cible du cerveau a été atteinte par la pointe de la sonde. A ce point, l'opération d'insertion est arrêtée, le stylet et/ou la canule sont retirés de la sonde et cette dernière est verrouillée à l'aide d'un dispositif de fixation susceptible d'assurer son maintien en place pendant une durée qui peut atteindre plusieurs années.

Le document « Medtronic - DBS™ Lead Kit for Deep Brain Stimulation 3387 3389 - Implant manual » de la société Medtronic Inc., téléchargeable du site Internet : http://www.medtronic.com/physician/activa/downloadablefiles/ 197928_b_006.pdf décrit des sondes pour neurostimulation électrique profonde et leur procédé d'implantation.

Ce document décrit également un dispositif de fixation comportant une pièce en forme de manchon destinée à être insérée dans une ouverture crânienne réalisée par craniotomie, et un capuchon susceptible de s'engager avec le manchon pour fermer de manière étanche ladite ouverture. La pièce en forme de manchon comporte une collerette destinée à reposer sur la surface extérieure du crâne du patient et présentant des rainures orientées radialement. Pour fixer une sonde implantée à l'aide d'un tel dispositif, il est nécessaire de plier d'environ 90° l'extrémité proximale (c'est à dire sortant du crâne) de ladite sonde et l'introduire dans l'une desdites rainures, où elle sera maintenue en place par le couvercle.

Dans ces conditions l'opération de fixation de la sonde est susceptible de provoquer des petits déplacements involontaires de sa pointe à l'intérieur du cerveau du patient. Sachant que les régions-cibles devant être stimulées ont des dimensions de l'ordre de quelques millimètres, ces déplacements peuvent réduire considérablement l'efficacité de la stimulation.

Le document US 7,302,298 décrit une sonde de stimulation cérébrale profonde maintenue en place par une vis introduite directement dans une ouverture pratiquée dans le crâne d'un patient. Cette méthode de fixation présente le grave défaut d'entraîner une dégradation rapide du revêtement de passivation de la sonde.

Le document US 6 321 104 montre un dispositif selon le préambule de la revendication 1.

L'invention vise à procurer un dispositif de fixation permettant de fixer de manière simple et reproductible une ou, de préférence, plusieurs sondes (par exemple, entre 1 et 10, et typiquement, 4 ou 5), dont le positionnement par rapport à des cibles de dimensions millimétrique est critique.

Plus particulièrement, l'invention vise à procurer un dispositif de fixation permettant de verrouiller rapidement une sonde lorsque son insertion est réputée satisfaisante, tout en évitant que l'opération de verrouillage de la sonde n'introduise des déplacements involontaires et incontrôlés de sa pointe.

Conformément à l'invention un tel but peut être atteint par un dispositif de fixation transcrânien pour sondes de stimulation cérébrale profonde comprenant : une pièce principale en forme de manchon ou de gobelet, destinée à être fixée à l'intérieur d'une ouverture pratiquée dans le crâne d'un patient; et une première et une deuxième pièce de fixation, superposées à l'intérieur de ladite pièce principale ; lesdites pièces de fixation comportant au moins un alésage axial respectif adapté pour permettre le passage d'une ou plusieurs sondes de stimulation cérébrale profonde ; **caractérisé en ce que** lesdites pièces de fixation sont agencées de manière à pouvoir passer d'une première position relative, dans laquelle leurs alésages axiaux respectifs sont alignés de manière à permettre un coulissement axial de la ou des sondes, à une deuxième position relative, dans laquelle lesdits alésages axiaux sont partiellement désalignés de manière à enserrer la ou lesdites sondes empêchant un tel coulissement.

Selon des modes de réalisation particuliers de l'invention :
- Au moins une desdites première et deuxième pièces de fixations peut être susceptible de passer de ladite première à ladite deuxième position par rotation ou roto-translation autour d'un axe parallèle auxdits alésages.
- Au moins une desdites première et deuxième pièces de fixations peut être susceptible de passer de ladite première à ladite deuxième position par translation dans une direction perpendiculaire auxdits alésages. Plus particulièrement, une pièce tournante excentrique peut être prévue pour translater la première ou la deuxième pièce de fixation dans ladite direction perpendiculaire aux alésages. Avantageusement, l'une desdites pièces de fixation peut présenter au moins un élément de serrage en forme de pointe ou dièdre, susceptible de venir en contact avec l'une desdites sondes, tandis que l'autre pièce de fixation peut présenter au moins un éléments d'appui disposé d'un côté opposé de la sonde par rapport à l'élément de serrage, de telle manière que, lorsque lesdites première et deuxième pièces de fixation se trouvent dans leur deuxième position, ladite ou chaque sonde se trouve pincée entre un élément de serrage et un élément d'appui correspondants.
- Le dispositif peut comporter également un moyen de verrouillage desdites pièces dans leur première position relative.
- Lesdites pièces de fixation peuvent comporter une pluralité d'alésages, chacun desquels est adapté pour permettre le passage d'une et une seule sonde de stimulation cérébrale profonde.

Un autre objet de l'invention est un système de stimulation cérébrale profonde comprenant : un dispositif de fixation tel que décrit ci-dessus et au moins une sonde de stimulation cérébrale profonde insérée dans un alésage axial dudit dispositif.

D'autres caractéristiques, détails et avantages de l'invention ressortiront à la lecture de la description faite en référence aux dessins annexés donnés à titre d'exemple et qui représentent, respectivement :
- Les figures 1A et 1B, deux vues en section d'un dispositif de fixation transcrânien pour sondes de stimulation cérébrale profonde ;
- La figure 1C, une vue en plan de la deuxième pièce de fixation du dispositif des figures 1A et 1B ;
- La figure 2, une vue en section d'un dispositif selon un mode de réalisation de l'invention ;
- Les figures 3A et 3B, deux vues en section d'un dispositif selon une première variante dudit mode de réalisation de l'invention, dans sa première et deuxième position respectivement ;
- Les figures 4A et 4B, deux vues en section d'un dispositif selon une deuxième variante dudit mode de réalisation de l'invention, dans sa première et deuxième position respectivement ;
- Les figures 4C et 4D, des vues en plan correspondantes aux figures 4A et 4B, respectivement ; et
- Les figures 4E et 4F, deux vues en section d'un dispositif selon une troisième variante dudit mode de réalisation, convenant particulièrement bien au serrage de sondes de petit diamètre, dans sa première et deuxième position respectivement ;

Le principe à la base des différents modes de réalisation de l'invention consiste à enserrer la ou les sondes devant être verrouillées dans un alésage axial.

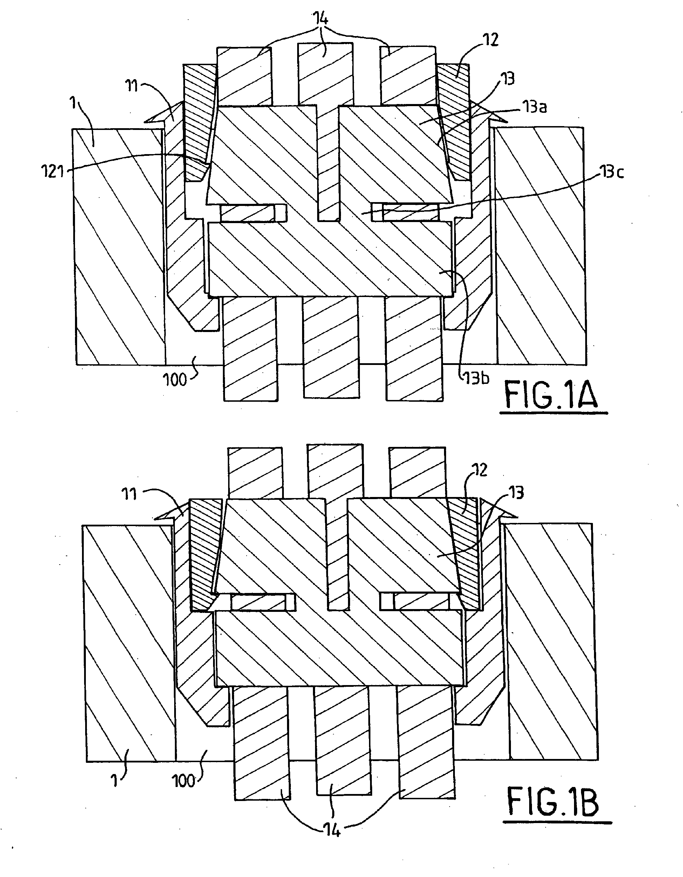

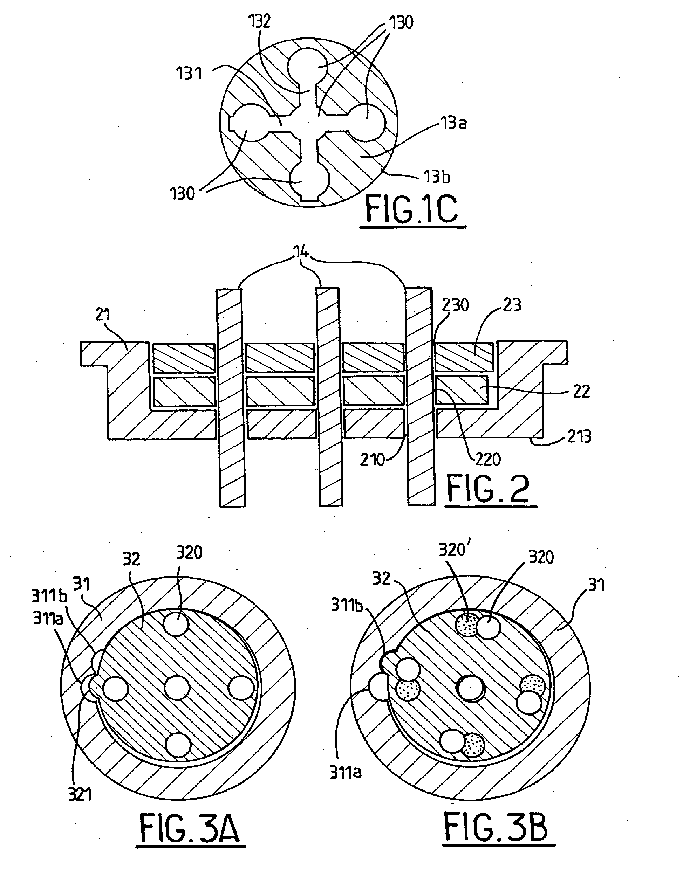

Les figures 1A, 1B et 1C illustrent un dispositif de fixation comportant une pièce dite principale 11, en forme de manchon, qui est destinée à être introduite dans une ouverture 100 pratiquée par craniotomie dans le crâne 1 d'un patient ; une première pièce de fixation 12 située du côté de la pièce en forme de manchon orienté vers l'extérieur du crâne 1 ; et une deuxième pièce de fixation 13 située du côté de la pièce en forme de manchon orienté vers l'intérieur du crâne 1. La première et la deuxième pièce de fixation ont des dimensions et des formes leur permettant d'être logées à l'intérieur de la pièce principale 11 en forme de manchon.

La première pièce de fixation 12 présente à son tour une forme de manchon, avec un alésage axial unique dont la surface intérieure est conique ou pyramidale. La surface externe de cette pièce présente une forme qui épouse celle de la surface interne de la pièce principale 11.

La deuxième pièce de fixation 13 présente une pluralité d'alésages axiaux 130, dont les axes sont parallèles à celui de l'alésage unique de la pièce principale 11. Chacun de ces alésages est adapté pour permettre le passage d'une et une seule sonde de stimulation cérébrale profonde 14 (bien qu'en variante on pourrait envisager que chaque alésage soit traversé par plusieurs sondes). La figure 1C montre que, dans l'exemple considéré, la pièce 13 comporte cinq alésages 130 disposés en forme de croix.

Plus précisément, dans le dispositif des figures 1A - 1C, la deuxième pièce de fixation 13 est constituée d'un premier segment 13a et un deuxième segment 13b, reliés entre eux par des liaisons élastiques 13c. Le premier segment 13a, orienté vers l'extérieur du crâne 1, est découpé en quatre parties ou quartiers par deux entailles 131, 132 qui traversent les alésages 130.

Le premier segment 13a présente une surface extérieure tronconique ou pyramidale, qui épouse la forme de la surface intérieure de la première pièce de fixation, de manière à permettre l'emboîtement réciproque de ces deux éléments.

Pour implanter un système électrostimulation cérébrale profonde utilisant le système de fixation des figures 1A - 1C on procède de la façon suivante.

Premièrement, un perçage 100 d'environ 8 mm de diamètre est réalisé par craniotomie dans la boîte crânienne 1 d'un patient, l'axe du perçage étant déterminé en fonction du repérage de la ou des cibles dans le cerveau dudit patient (par exemple, le noyau subthalamique pour la maladie de Parkinson). La pièce principale 11 en forme de manchon est insérée dans ce perçage, une collerette étant prévue pour servir de butée à l'enfoncement. Le positionnement angulaire de ladite pièce principale à l'intérieur du perçage peut être assuré par une vis s'insérant dans la collerette à l'instar d'une butée angulaire ou d'un pion de centrage. Alternativement, la position angulaire de la pièce 11 peut également être verrouillée par collage ou par précontrainte.

Ensuite, la ou les cibles sont repérées de manière plus précise par imagerie électrophysiologie avec des sondes per-opératoires. La deuxième pièce de fixation 13 est introduite dans le manchon 11, jusqu'à se positionner sur un siège prévu à cet effet ; puis c'est la première pièce de fixation 12 qui est positionnée. A ce stade, on veillera à ne pas emboîter de force les deux pièces de fixation.

Une ou, de préférence, plusieurs sondes de stimulation (cinq dans l'exemple) sont placées dans un robot d'implantation pour être glissées dans les alésages 130 de la pièce 13.

Lorsque la profondeur d'enfoncement des sondes correspond à la position des cibles repérées avec les sondes d'exploration électrophysiologique, le verrouillage est réalisé en faisant descendre la première pièce 12 de manière à emboîter les deux pièces de fixation 12 et 13. Ainsi, la pression radiale exercée par la paroi intérieure conique de la première pièce de verrouillage 12 provoque le rapprochement de différentes parties constituant le premier segment 13a de la deuxième pièce de fixation. De cette manière, les alésages 130 de ladite deuxième pièce de fixation se resserrent autour des sondes 14, qui sont ainsi maintenue fermement.

A la fin de la descente, le cliquet 121 solidarise de manière irréversible l'ensemble. En variante, ce cliquet peut ne pas être prévu : de cette manière l'assemblage est maintenu de manière réversible par les seules forces de frottement.

Les pièces 11, 12 et 13 présentent de préférence une forme à symétrie cylindrique. Elles peuvent ainsi être réalisées essentiellement par tournage.

A titre d'exemple, la pièce principale 11 en forme de manchon peut avoir un diamètre externe de 8 mm, un diamètre interne de 6 mm et une hauteur de 6 mm, avec des sièges prévus à 5 mm (pour la deuxième pièce de fixation) et à 3 mm (pour la première pièce de fixation) de son bord situé à l'extérieur du crâne. Dans ce cas, la deuxième pièce de fixation 13 peut être constituée d'un premier segment tronconique 13a ayant une hauteur de 2,5 mm, un diamètre de la plus grande base de 6 mm et un diamètre de la plus petite base de 5,4 mm ; le deuxième segment 13b peut avoir une forme cylindrique avec une hauteur de 2 mm et un diamètre de 6 mm ; et les éléments de liaison élastique 13b peuvent avoir une hauteur de 0,5 mm. Le diamètre des alésages 130, avant verrouillage, peut être typiquement de 1,45+/-0,1 mm. Ce diamètre est légèrement supérieur à celui des sondes normalement utilisées en stimulation cérébrale profonde (1,27 mm dans le cas des sondes Medtronic - marque déposée) ; en effet, avant l'introduction des sondes de stimulation, il est nécessaire de réaliser une exploration électrophysiologique des cibles, ce qui se fait au moyen de microsondes introduites à travers des canules ayant un diamètre d'environ 1,45mm. Les perçages des pièces de fixation doivent être compatibles avec ces canules.

Les matériaux employés doivent satisfaire les contraintes de biocompatibilité pour une implantation de plus de 28 jours. Il peut s'agir par exemple de PTFE (polytétrafluoroéthylène) ou du PEEK (polyétheréthercétone) de grade médical. Des métaux biocompatibles existent mais ne sont pas privilégiés car ils peuvent générer des artéfacts sur les images de résonance magnétique nucléaire ; en outre, à cause de leur rigidité des efforts relativement importants seraient nécessaires pour mettre en oeuvre l'opération de verrouillage des sondes.

La figure 2 montre un schéma de principe d'un mode de réalisation de l'invention, qui se décline à son tour en une pluralité de variantes. Dans ce mode de réalisation, une première pièce de fixation 22 et une deuxième pièce de fixation 23, globalement en forme de plaque ou de disque, sont superposées à l'intérieur d'une pièce principale 21 en forme de gobelet. Les deux pièces de fixation présentent un ou plusieurs alésages axiaux respectifs (220, 230) qui sont alignés lorsque lesdites pièces se trouvent dans une première position relative, et partiellement désalignés lorsque lesdites pièces se trouvent dans une deuxième position. On comprend que lorsque les alésages sont alignés, les sondes 14 peuvent coulisser librement, tandis qu'en cas de désalignement elles se trouvent pincées entre les pièces de fixation, et donc verrouillées en position.

Dans l'exemple de la figure 2 la pièce principale 21 est définie « en forme de gobelet » car elle présente un fond 213, orienté vers l'intérieur du crane du patient et traversé à son tour par des alésages axiaux 210. En variante, cette pièce pourrait avoir une forme de manchon avec un rebord interne destiné à maintenir les pièces de fixation en place. Cependant, l'utilisation d'une pièce principale en forme de gobelet est préférée, car les alésages 210 contribuent au maintien des sondes de stimulation 14.

Comme dans le cas des figures 1A - 1C, les matériaux employés doivent satisfaire les contraintes de biocompatibilité pour une implantation de plus de 28 jours. Il peut s'agir par exemple de PTFE (polytétrafluoroéthylène) ou du PEEK (polyétheréthercétone) de grade médical.

Les figures 3A et 3B illustrent une première variante d'un dispositif selon ce mode de réalisation de l'invention.

Dans ces figures, les références 31 et 32 indiquent la pièce principale et la première pièce de fixation, respectivement ; la deuxième pièce de fixation n'est pas visible, car elle se trouve au-dessous de la pièce 32.

Les deux pièces de fixation ont la forme d'un disque, et comportent chacune cinq alésages axiaux 320 dont le diamètre est adapté pour permettre le passage d'une sonde de stimulation. La deuxième pièce est solidaire de la pièce principale ; à la limite, ces deux pièces pourraient être réalisées d'un seul tenant. Au contraire, la première pièce de fixation 32 peut se déplacer à l'intérieur de la pièce principale par un mouvement de roto-translation - l'amplitude de la rotation étant typiquement de 5° à 20° - afin de désaligner ses alésages 320 de ceux (non représentés) de la deuxième pièce de fixation. S'il n'y avait pas d'alésage en position centrale, un mouvement de rotation pure pourrait être suffisant.

On remarquera que la sonde centrale, lorsqu'elle est présente, est nécessairement moins serrée que les autres. Par conséquent, cette variante de réalisation convient plutôt à une configuration à quatre alésages et à quatre sondes.

La figure 3A représente le dispositif de fixation lorsque les alésages des différentes pièces sont alignés ; sur la figure 3B, les références 320 indiquent les alésages en position de désalignement, tandis que les références 320' montrent, par comparaison, ces mêmes alésages en position d'alignement.

Une saillie radiale ou ergot 321 prévue sur la surface extérieure de la pièce 32 et deux encoches 311 a, 311b de dimensions différentes (dans l'exemple des figures, l'encoche 311 b est sensiblement plus petite que l'encoche 311 a) sur la surface intérieure de la pièce principale 31 permettent de verrouiller, de manière réversible, la deuxième pièce de fixation dans ses deux positions extrêmes.

En effet, dans ces conditions, un mouvement de rotation de 32 par rapport à 31 amenant l'ergot 321 de la « grande » encoche 311 a à la « petite » encoche 311 b entraîne une translation de la pièce 32 dans un plan perpendiculaire à l'axe des alésages 320. Une telle translation présente une amplitude de quelques centaines de µm, typiquement 350 µm environ. Son effet est de contraindre la pièce de fixation 32 et de pincer les sondes à l'intérieur des alésages.

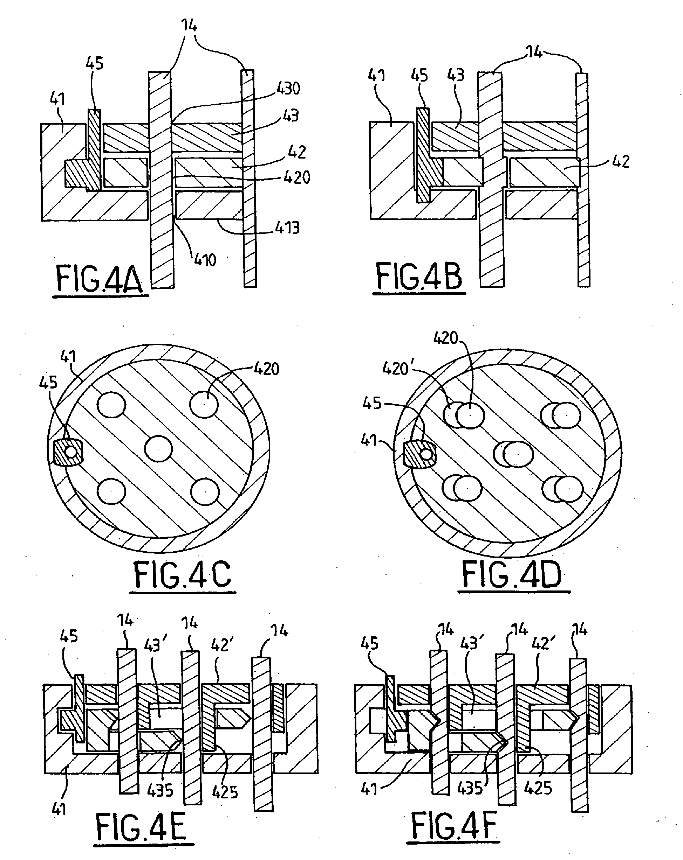

Selon une deuxième variante du mode de réalisation de l'invention, illustrée par les figures 4A à 4D, le désalignement des alésages axiaux qui bloque en place les sondes de stimulation est produit par un mouvement de translation relative des deux pièces de fixation. Il s'agit là de la variante que semble offrir les meilleures performances.

Le dispositif des figures 4A à 4D comporte une pièce principale 41 en forme de gobelet pourvue d'un fond 413, ainsi qu'une première et une deuxième pièce de fixation superposées (références 42 et 43). Ces trois éléments présentent des alésages axiaux 410, 420 et 430 qui, dans une première position, sont alignés afin de permettre le coulissement des sondes de stimulation 14, et dans une deuxième position sont désalignés de manière à verrouiller lesdites sondes. Plus précisément, l'excentration est produite par un déplacement de la première pièce de fixation dans une direction perpendiculaire à l'axe des alésages. Ce déplacement peut être provoqué par la rotation d'une pièce excentrique ou ergot 45. Avantageusement, la partie supérieure de cet ergot peut être façonnée de manière à pouvoir recevoir facilement un outil destiné à la faire tourner ; par exemple elle peut comporter une rainure destinée à recevoir la lame d'un tournevis.

Les figures 4A et 4B montrent une vue partielle en coupe du dispositif dans sa première et sa deuxième position, respectivement. Les figures 4C et 4D montrent une vue en plan de ce dispositif, également dans sa première et sa deuxième position, la deuxième pièce de fixation ayant été enlevée pour rendre visible la première pièce 42. On peut remarquer que la première pièce de fixation présente un méplat sur lequel agit l'ergot 45, et que son diamètre est inférieur à celui de la pièce principale 41 afin d'autoriser le jeu nécessaire au mouvement de translation. La géométrie de cette pièce est telle que les positions « sondes serrées » ou « sondes libres » correspondent à deux positions d'équilibre mécanique. Compte tenu des efforts en torsion appliquée à la pièce 45, elle sera préférentiellement réalisée en métal biocompatible (titane ou inox de grade médical).

Le dispositif des figures 4A - 4D convient tout particulièrement au serrage de sondes ayant un diamètre supérieur à 1 mm, et typiquement compris entre 1 et 1,5 mm. Pour des sondes de plus petites dimensions (diamètre compris entre 300 µm et 1 mm environ) il est préférable d'utiliser la variante représentée sur les figures 4E (position libre) et 4F (position de serrage). Dans un tel dispositif, la deuxième pièce de serrage 43' comporte des éléments de serrage 435 en forme de pointe ou dièdre, et la première pièce 42' des éléments d'appui en forme de cloison 425, s'étendant dans une direction verticale (c'est à dire parallèlement à la direction de coulissement des sondes). Les éléments de serrage 435 sont agencés pour comprimer les sondes 14 contre les éléments d'appui correspondants, qui fonctionnent comme des enclumes. Ainsi, les sondes sont fixées par pincement localisé. On peut également envisager un agencement complémentaire, dans lequel les éléments de serrage seraient portés par la première pièce de fixation et les éléments d'appui par la deuxième pièce.

On remarquera que les éléments de serrage 435 pour les différentes sondes peuvent être situés sur des plans différents.

Tous les dispositifs représentés dans les figures et décrits en détails ci-dessus sont adaptés à la fixation de cinq sondes, mais cela ne constitue nullement une limitation de l'invention. Le nombre de sondes peut être compris entre 1 et 10, voire davantage. L'utilisation de quatre ou cinq sondes paraît particulièrement préférable.

## Revendications

1. Dispositif de fixation transcrânien pour sondes de stimulation cérébrale profonde comprenant :
- une pièce principale (21, 31, 41, 51) en forme de manchon ou de gobelet, destinée à être fixée à l'intérieur d'une ouverture (100) pratiquée dans le crâne d'un patient (1) ; et
- une première (22, 32, 42, 52) et une deuxième (13, 23, 43, 53) pièce de fixation, superposées à l'intérieur de ladite pièce principale ;
lesdites pièces de fixation comportant au moins un alésage axial respectif (220, 230, 320, 420, 430) adapté pour permettre le passage d'une ou plusieurs sondes de stimulation cérébrale profonde (14) ;
**caractérisé en ce que** lesdites pièces de fixation sont agencées de manière à pouvoir passer d'une première position relative, dans laquelle leurs alésages axiaux respectifs sont alignés de manière à permettre un coulissement axial de la ou des sondes, à une deuxième position relative, dans laquelle lesdits alésages axiaux sont partiellement désalignés de manière à enserrer la ou lesdites sondes empêchant un tel coulissement.

2. Dispositif selon la revendication 1, dans lequel au moins une desdites première et deuxième pièces de fixations est susceptible de passer de ladite première à ladite deuxième position par rotation ou roto-translation autour d'un axe parallèle auxdits alésages.

3. Dispositif selon la revendication 1, dans lequel au moins une desdites première et deuxième pièces de fixations est susceptible de passer de ladite première à ladite deuxième position par translation dans une direction perpendiculaire auxdits alésages.

4. Dispositif selon la revendication 3, dans lequel une pièce tournante excentrique (45) est prévue pour translater la première ou la deuxième pièce de fixation dans ladite direction perpendiculaire aux alésages.

5. Dispositif selon l'une des revendications 3 et 4, dans lequel l'une desdites pièces de fixation (43') présente au moins un élément de serrage (430) en forme de pointe ou dièdre, susceptible de venir en contact avec l'une desdites sondes, tandis que l'autre pièce de fixation (42') présente au moins un éléments d'appui (420) disposé d'un côté opposé de la sonde (14) par rapport à l'élément de serrage, de telle manière que, lorsque lesdites première et deuxième pièces de fixation se trouvent dans leur deuxième position, ladite ou chaque sonde se trouve pincée entre un élément de serrage et un élément d'appui correspondants.

6. Dispositif selon l'une des revendications précédentes, comportant également un moyen de verrouillage (311a, 311 b, 321) desdites pièces dans leur première position relative.

7. Dispositif selon l'une des revendications précédentes, dans lequel lesdites pièces de fixation comportent une pluralité d'alésages, chacun desquels est adapté pour permettre le passage d'une et une seule sonde de stimulation cérébrale profonde.

8. Système de stimulation cérébrale profonde comprenant :
- un dispositif de fixation selon l'une des revendications précédentes ; et
- au moins une sonde de stimulation cérébrale profonde (14) insérée dans un alésage axial dudit dispositif.

## Patentansprüche

1. Vorrichtung zur transkraniellen Befestigung für Sonden zur tiefen cerebralen Stimulation, umfassend:
- ein Hauptteil (21, 31, 41, 51) in Form eines Stutzens oder eines Bechers, welches dazu bestimmt ist, im Inneren einer Öffnung (100) befestigt zu werden, welche in dem Schädel eines Patienten (1) hergestellt ist; und
- ein erstes (22, 32, 42, 52) und ein zweites (13, 23, 43, 53) Befestigungsteil, welche im Inneren des Hauptteils überlagert sind;
wobei die Befestigungsteile jeweils wenigstens eine axiale Bohrung (220, 230, 320, 420, 430) umfassen, welche dazu ausgestaltet ist, den Durchgang einer oder mehrerer Sonden zur tiefen cerebralen Stimulation (14) zu ermöglichen;
**dadurch gekennzeichnet, dass** die Befestigungsteile dazu ausgestaltet sind, von einer ersten relativen Position, in welcher ihre jeweiligen axialen Bohrungen derart ausgerichtet sind, dass sie ein axiales Gleiten der Sonde oder der Sonden erlauben, in eine zweite relative Position, in welcher die axialen Bohrungen teilweise derart versetzt sind, dass sie die Sonde oder Sonden einspannen, wodurch ein solches Gleiten verhindert wird, übergehen zu können.

2. Vorrichtung nach Anspruch 1, wobei wenigstens eines von dem ersten und zweiten Teil zur Befestigung dazu geeignet ist, durch eine Drehung oder Drehverschiebung um eine Achse parallel zu den Bohrungen aus der ersten in die zweite Position überzugehen.

3. Vorrichtung nach Anspruch 1, wobei wenigstens eines von dem ersten und zweiten Teil zur Befestigung dazu geeignet ist, durch eine Verschiebung in einer Richtung senkrecht zu den Bohrungen aus der ersten in die zweite Position überzughen.

4. Vorrichtung nach Anspruch 3, wobei ein exzentrisches Drehstück (45) vorgesehen ist, um das erste oder das zweite Teil zur Befestigung in der Richtung senkrecht zu den Bohrungen zu verschieben.

5. Vorrichtung nach einem der Ansprüche 3 und 4, wobei eines von den Teilen zur Befestigung (43') wenigstens ein Einspannelement (430) in Form einer Spitze oder eines Dieders umfasst, welches dazu geeignet ist, mit einer der Sonden in Kontakt zu kommen, während das andere Teil zur Befestigung (42') wenigstens ein Anschlagelement (420) aufweist, welches auf einer Seite gegenüber der Sonde (14) bezüglich des Einfasselements derart angeordnet ist, dass wenn das erste und zweite Element zur Befestigung sich in ihrer zweiten Position befinden, die oder jede Sonde zwischen einem Einfasselement und einem Anschlagelement, die einander entsprechen, eingeklemmt ist.

6. Vorrichtung nach einem der vorhergehenden Ansprüche, umfassend außerdem ein Verriegelungsmittel (311a, 311b, 321) für die Teile in ihrer ersten relativen Position.

7. Vorrichtung nach einem der vorhergehenden Ansprüche, wobei die Teile zur Befestigung eine Vielzahl von Bohrungen umfassen, von welchen jede dazu ausgestaltet ist, den Durchgang von nur einer Sonde zur tiefen cerebralen Stimulation zu ermöglichen.

8. System zur tiefen cerebralen Stimulation, umfassend:
- eine Vorrichtung zur Befestigung nach einem der vorhergehenden Ansprüche; und
- wenigstens eine Sonde zur tiefen cerebralen Stimulation (14), welche in eine axialen Bohrung der Vorrichtung eingesetzt ist.

## Claims

1. A transcranial securing device for deep brain stimulation leads, comprising:
• a principal part (21, 31, 41, 51) in the form of a sleeve or cup for securing inside an opening formed in the skull of a patient; and
• a first (22, 32, 42, 52) and a second (13, 23, 43, 53)securing part superimposed inside said principal part;
• said securing parts respectively comprising at least one axial bore (220, 230, 320, 420, 430) adapted to allow the passage of one or more deep brain stimulation leads (14);
**characterised in that** said securing parts are arranged so as to be capable of passing from a first relative position in which their respective axial bores are aligned to allow the lead or leads to slide axially, to a second relative position in which said axial bores are partially misaligned in order to grip the lead or leads, preventing said sliding.

2. A device according to claim 1, wherein at least one of said first and second securing parts is capable of passing from said first to said second position by rotation or rotation-translation about an axis parallel to said bores.

3. A device according to claim 1, wherein at least one of said first and second securing parts is capable of passing from said first to said second position by translation in a direction perpendicular to said bores.

4. A device according to claim 3, wherein an eccentric turning part (45) is provided in order to move the first or the second securing part in translation in said direction perpendicular to the bores.

5. A device according to claim 3 or claim 4, wherein one of said securing parts (43') has at least one gripping element (430) in the form of a tip or dihedral, which is capable of coming into contact with one of said leads, while the other securing part (42') has at least one support element(420) disposed on an opposite side of the lead (14) relative to the gripping element, such that when said first and second securing parts are in their second position, said or each lead is clamped between a corresponding gripping element and a support element.

6. A device according to any of the preceding claims, also comprising a means for locking (311a, 311b, 321) said parts in their first relative position.

7. A device according to any of the preceding claims, wherein said securing parts comprise a plurality of bores, each of which is adapted to allow the passage of one and only one deep brain stimulation lead.

8. A deep brain stimulation system comprising:
• a securing device according to any of the preceding claims; and
• at least one deep brain stimulation lead inserted into an axial bore of said device.
